# EUROPEAN PATENT SPECIFICATION

(11) **EP 2 950 702 B1**
(45) Date of publication and mention of the grant of the patent: **16.08.2017**
(21) Application number: 14746806.0
(22) Date of filing: 24.01.2014
(51) Int. Cl.: A61B 1/00, A61B 1/04, A61B 1/05

(54) **ENDOSCOPE**
ENDOSKOP
ENDOSCOPE

(30) Priority: 29.01.2013 US 201361757791 P
(43) Date of publication of application: 09.12.2015
(73) Proprietor: Olympus Corporation, Tokyo 192-8507 (JP)
(72) Inventor: KISHI, Kosuke, Hachioji-shi, Tokyo 192-8507 (JP); MURATA, Takeshi, Hachioji-shi, Tokyo 192-8507 (JP)
(74) Representative: Schicker, Silvia
(86) International application number: PCT/JP2014/052176
(87) International publication number: WO 2014/119697

(56) References cited:
- WO-A1-2012/046413
- WO-A2-2012/016224
- JP-A- S 516 739
- JP-A- 2008 220 709
- JP-A- 2013 009 896
- US-A1- 2002 103 420
- US-A1- 2003 032 863
- US-A1- 2010 030 031
- US-A1- 2012 059 222

## Description

### Technical Field

The present invention relates to an endoscope.

Priority is claimed on US Provisional Patent Application No. 61/757,791, filed January 29, 2013, the content of which is incorporated herein by reference.

### Background Art

In the related art, an endoscope including an imaging part disposed at a distal end of an endoscope and configured to image an external imaging target through an observation window is known. For example, in Patent Literature 1, an endoscope in which a field of vision range of the imaging part is changed as the imaging part is moved with respect to the observation window is disclosed.

Furthermore, in the related art, an endoscope including a nozzle configured to spray a cleaning liquid or a gas toward an observation window is known. In addition, an endoscope in which a nozzle configured to spray a cleaning liquid or a gas is controlled to move toward foulings is known (for example, Patent Literature 2). Document US 2010/030031 A1 discloses a variable viewing endoscope as set out in the preamble of claim 1.

### Citation List

### Patent Literature

Patent Literature 1: Japanese Unexamined Patent Application, First Publication No. 2012-75778
Patent Literature 2: Japanese Unexamined Patent Application, First Publication No. 2008-220709

### Summary of Invention

### Technical Problem

In the endoscope of the related art, since the nozzle is moved toward the foulings, even a place not related to a direction in which an operator wants to look may be cleaned merely because there are contaminants adhered thereto. In this case, useless cleanings may be repeated, thus increasing consumption of cleaning water and easily decreasing the efficiency of the cleaning.

In consideration of the above-mentioned circumstances, the present invention is directed to provide an endoscope capable of cleaning an observation window in a direction to be imaged by an imaging part, and performing effective cleaning. Solution to Problem

An endoscope according to a first aspect of the present invention includes a distal end housing which is provided at a distal end of an endoscope insertion unit; an observation window which is provided at the distal end housing and configured to be capable of observing an outside of the distal end housing from the inside of the distal end housing; an imaging part which is provided inside the distal end housing and configured to image an imaging target which is positioned outside the distal end housing through the observation window; an imaging part moving unit which is configured to move the imaging part with respect to the observation window and to change a range of an imaging view field of the imaging part; a cleaning unit configured to clean an outer surface of the observation window; and a synchronizing unit which is configured to synchronize a change operation of a rang of an imaging view field of the imaging part by the imaging part moving unit with a cleaning operation by the cleaning unit.

According to a second aspect of the present invention, the endoscope according to the first aspect may further include a cleaning position change unit which is configured to change a cleaning position of the cleaning unit with to be cleaned by the cleaning unit. The synchronizing unit may synchronize the change operation changing a rang of the imaging view field of the imaging part by the imaging part moving unit with a change operation changing the cleaning position by the cleaning position change unit.

According to a third aspect of the present invention, in the endoscope according to the second aspect, the cleaning position change unit may use a driving force in common with the imaging part moving unit by being be connected to the imaging part moving unit via a power transmission unit serving as the synchronizing unit.

According to a fourth aspect of the present invention, in the endoscope according to the third aspect, the cleaning position change unit may be connected to the imaging part moving unit via the power transmission such that the cleaning position is changed to a position of the observation window including an imaging view field direction of the imaging part which is moved by the imaging part moving unit.

According to a fifth aspect of the present invention, in the endoscope according to the third aspect, the cleaning position change unit may be able to change a cleaning position of the observation window to a position which is different from an imaging view field direction of the imaging part which is moved by the imaging part moving unit and the cleaning position change unit is connected to the imaging part moving unit via the power transmission unit

According to a sixth aspect of the present invention, in the endoscope according to the second aspect, the cleaning unit may have a plurality of regional cleaning units configured to clean a plurality of regions of the outer surface of the observation window. The cleaning position change unit may change the cleaning position by selecting at least one regional cleaning unit from the plurality of regional cleaning units.

According to a seventh aspect of the present invention, in the endoscope according to the second aspect, the part moving unit may have a first driving unit and the cleaning position change unit may have a second driving unit. The synchronizing unit may be a control unit which is configured to synchronize the first driving unit with the second driving unit.

According to an eighth aspect of the present invention, in the endoscope according to the first aspect, the cleaning unit may include an injection nozzle configured to spray a cleaning fluid toward the outer surface of the observation window.

According to a ninth aspect of the present invention, in the endoscope according to the eighth aspect, the synchronizing unit may be a control unit configured to perform a spray control of the fluid from the injection nozzle so as to be synchronized with the change operation of a range of the imaging view field of the imaging part by the imaging part moving unit.

According to a tenth aspect of the present invention, in the endoscope according to the second aspect, the cleaning unit may include an injection nozzle configured to spray a cleaning fluid toward the outer surface of the observation window, and the cleaning position change unit may change a cleaning position by moving a direction of the injection nozzle.

According to an eleventh aspect of the present invention, in the endoscope according to the eighth aspect, the cleaning unit may include an injection nozzle configured to spray a cleaning fluid toward the outer surface of the observation window. The cleaning position change unit may have a reflective plate configured to reflect the cleaning fluid which is sprayed from the injection nozzle toward the outer surface of the observation window, and change a cleaning position using the cleaning fluid by moving a direction of the reflective plate is moved.

### Advantageous Effects of Invention

According to the endoscope, the observation window can be cleaned at a position including a direction to be imaged by the imaging part, and the cleaning can be efficiently performed. According to the endoscope, since a part of the observation window at which an outside area of the imaging view field of the imaging part which is not necessary to clean does not sprayed the cleaning water, the cleaning can be efficiently performed.

### Brief Description of Drawings

Fig. 1 is a perspective view schematically showing a configuration of a distal end of an endoscope according to a first embodiment of the present invention.
Fig. 2 is a flowchart showing a cleaning control flow performed in the endoscope according to the first embodiment of the present invention.
Fig. 3 is a timing chart of a cleaning operation performed in the endoscope according to the first embodiment of the present invention.
Fig. 4 is a perspective view schematically showing a configuration of a distal end of an endoscope according to a second embodiment of the present invention.
Fig. 5 is a plan view of a partial configuration of the endoscope according to the second embodiment of the present invention.
Fig. 6 is a flowchart showing a cleaning control flow performed in the endoscope of the second embodiment of the present invention.
Fig. 7 is a timing chart of a cleaning operation performed in the endoscope according to the second embodiment of the present invention.
Fig. 8 is a plan view showing a configuration of a cleaning unit and a cleaning position change unit included in an endoscope according to a third embodiment of the present invention.
Fig. 9 is a front view showing the configuration of the cleaning unit and the cleaning position change unit included in the endoscope according to the third embodiment of the present invention.
Fig. 10 is a perspective view schematically showing a configuration of a distal end of an endoscope according to a fourth embodiment of the present invention.
Fig. 11 is a perspective view schematically showing a configuration of a distal end of an endoscope according to a fifth embodiment of the present invention.
Fig. 12 is a block diagram showing a configuration of a driving system of the endoscope according to the fifth embodiment of the present invention.
Fig. 13 is a flowchart showing the cleaning control flow performed in the endoscope according to the fifth embodiment of the present invention.
Fig. 14 is a timing chart of a cleaning operation performed in the endoscope according to the fifth embodiment of the present invention.
Fig. 15 is a timing chart of another cleaning operation performed in the endoscope according to the fifth embodiment of the present invention.
Fig. 16 is a timing chart of another cleaning operation performed in the endoscope according to the fifth embodiment of the present invention.
Fig. 17 is a timing chart of another cleaning operation performed in the endoscope according to the fifth embodiment of the present invention.
Fig. 18 is a timing chart of another cleaning operation performed in the endoscope according to the fifth embodiment of the present invention.
Fig. 19 is a timing chart of another cleaning operation performed in the endoscope according to the fifth embodiment of the present invention.

### Description of Embodiments

### (First embodiment)

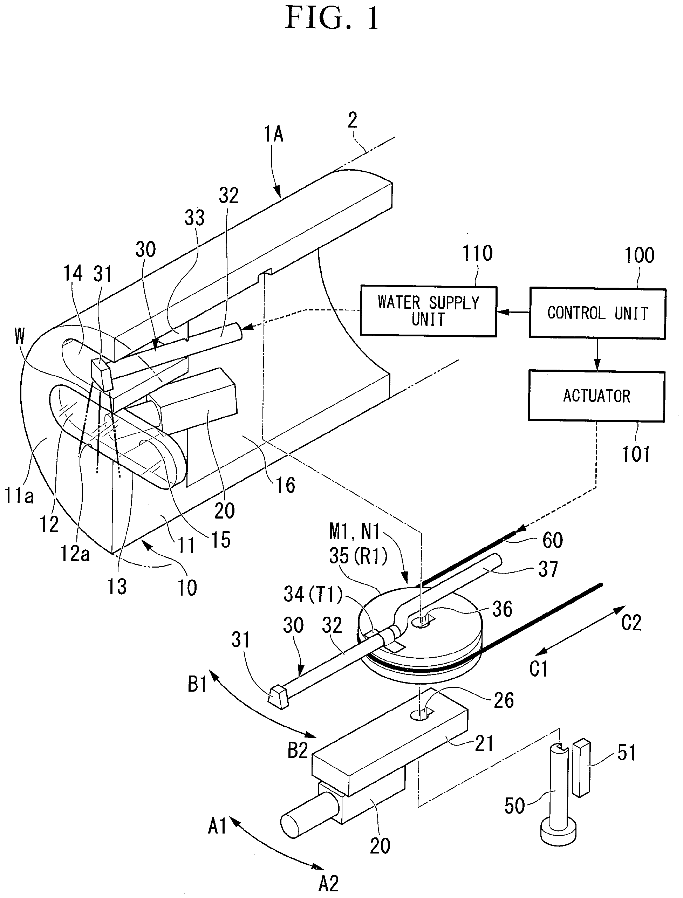

Hereinafter, an endoscope according to a first embodiment of the present invention will be described with reference to Figs. 1 to 3.

Fig. 1 is a perspective view schematically showing a configuration of a distal end of the endoscope 1A according to the first embodiment. The endoscope 1A according to the embodiment is a medical endoscope. As shown in Fig. 1, the endoscope 1A includes an insertion unit 2 inserted into a body from a distal end thereof. A distal end housing 10 formed of a hard material is provided at a distal end of the insertion unit 2 of the endoscope 1A. In the description, in the endoscope 1A, a side at which the distal end housing 10 is provided is referred to as a distal end, and a side at which a manipulation unit (not shown) is provided, i.e., a front side when the operator inserts the insertion unit 2 into the body, is referred to as a proximal end.

The distal end housing 10 has a columnar shape in which an axial direction is set to a longitudinal axis direction of the insertion unit 2. A housing space 16 is formed inside of a proximal end side of the distal end housing 10. A distal end side of the housing space 16 is closed by a distal end wall 11. A distal end surface 11 a of the distal end wall 11 is a flat surface perpendicular to the axial direction of the distal end housing 10. An observation window 12 is provided at the distal end surface 11a of the distal end wall 11. The observation window 12 is configured to enable observation of the outside of the distal end housing 10 from the inside of the distal end housing 10.

The observation window 12 is a window at which a cover 13 having optical transparency is provided. In the embodiment, the cover 13 has a transparent plate shape having an elliptical profile. An elliptical opening section 12a into which the cover 13 is fitted is formed at the observation window 12.

The elliptical opening section 12a is an opening section having an elliptical transverse shape in a cross section perpendicular to the axial direction of the distal end housing 10. The transparent cover 13 is fitted into the elliptical opening section 12a such that an outer surface thereof and the distal end surface 11a of the distal end housing 10 are flush. The cover 13 is fixed to the opening section 12a. The elliptical opening section 12a is formed such that a long diameter direction of an ellipse is arranged along a radial direction of the distal end surface 11a at a position adjacent to a center of the circular distal end surface 11a of the distal end wall 11.

The cover 13 fixed to the opening section 12a closes the opening section 12a such that no fluid or solid matter is inserted from the outside into the distal end housing 10 through the observation window 12. In addition, the cover 13 is formed to pass light therethrough such that an imaging part 20 can observe an observation target of the outside via the observation window 12.

A cavity section 15 is formed inside the observation window 12 (the distal end housing 20). In the cavity section 15, a distal end side comes in communication with the elliptical opening section 12a and a proximal end side comes in communication with the housing space 16. The imaging part 20 is accommodated in the cavity section 15. The imaging part 20 images the imaging target outside the distal end housing 10 through the observation window 12. For example, the imaging part 20 includes an imaging sensor provided with an imaging element, a camera control unit configured to control the imaging sensor, an optical system configured to collect light on the imaging sensor, and so on.

The imaging part 20 is attached to a support member 21 in the cavity section 15. Accordingly, the imaging part 20 is held in the cavity section 15 while not in contact with an inner wall of the cavity section 15.

A proximal end side of the support member 21 is disposed in the housing space 16. The proximal end side of the support member 21 is connected to a pulley 35 in the housing space 16. Specifically, a connecting shaft 50 and a shaft hole 26 are formed at the proximal end side of the support member 21. The connecting shaft 50 is connected to the pulley 35. A key 51 is inserted into the shaft hole 26. The connecting shaft 50 and the key 51 are fitted into a shaft hole 36 of the pulley 35 and the shaft hole 26 of the support member 21. The support member 21 is connected to the pulley 35 by the connecting shaft 50 and the key 51 such that the support member 21, the shaft hole 36, the connecting shaft 50 and the key 51 are integrally rotated about the connecting shaft 50.

The proximal end side portion of the support member 21, the pulley 35, and the connecting shaft 50 are accommodated in the housing space 16 of the proximal end side of the distal end housing 10. Then, in the housing space 16, the connecting shaft 50 is attached to the distal end housing 10 to be rotatable with respect to the distal end housing 10. A direction of a rotational axis of the connecting shaft 50 is perpendicular to the axial direction of the distal end housing 10. The direction of the rotational axis of the connecting shaft 50 is perpendicular to a long diameter direction of an ellipse of the elliptical opening section 12a constituting the observation window 12 (hereinafter, the direction is referred to as "a long diameter direction of the observation window 12"). In addition, the rotational axis of the connecting shaft 50 is disposed at a position passing a center of a circular cross section of the distal end housing 10. Accordingly, the imaging part 20 attached to the support member 21 is provided in a surface in the long diameter direction of the observation window 12 to be swung in directions of arrows A1 and A2 about the connecting shaft 50. A swing center of the imaging part 20 is disposed on a diameter of the circular cross section of the distal end housing 10.

The pulley 35 has a circular plate shape in which the shaft hole 36 is formed. The connecting shaft 50 and the key 51 are inserted into the shaft hole 36. A cleaning pipe 32 of a cleaning unit 30 (to be described later) is fixed to the pulley 35. A manipulation wire 60 is hung on an outer circumferential surface of the pulley 35. The manipulation wire 60 extends from the proximal end side (the manipulation unit side) of the insertion unit 2 of the endoscope 1A toward the distal end side, is wound on the pulley 35 to a half turn, and then is returned toward the proximal end side from the distal end side of the insertion unit 2. The manipulation wire 60 is operated by an actuator 101 (to be described later) to be pushed and pulled in directions of arrows C1 and C2. Then, as the manipulation wire is pushed and pulled in the directions of the arrows C1 and C2, the pulley 35 is pivoted. Then, when the pulley 35 is pivoted, the cleaning pipe 32 fixed to the pulley 35 is integrally pivoted with the pulley 35.

In addition, a force transmitted from the manipulation wire 60 to the pulley 35 is transmitted to the support member 21 via the connecting shaft 50 and the key 51. Accordingly, the support member 21 and the imaging part 20 are integrally pivoted with the pulley 35.

In the embodiment, the imaging part 20 and the cleaning pipe 32 of the cleaning unit 30 are configured to be integrally pivoted by pushing and pulling of the manipulation wire 60. That is, in the embodiment, the imaging part 20 and the cleaning unit 30 are performed in conjunction with each other.

The actuator 101 configured to push and pull the manipulation wire 60 is provided at the proximal end side of the insertion unit 2. An operation of the actuator 101 is controlled by a control unit 100 disposed at the proximal end side of the insertion unit 2. The operator may push or pull the manipulation wire 60 via a knob or a lever instead of the actuator 101.

The cleaning unit 30 is provided at the distal end housing 10, and has an injection nozzle 31 and the cleaning pipe 32. The injection nozzle 31 is a conduit member with one end in communication with the cleaning pipe 32 and the other end opened toward the cover 13. The injection nozzle 31 is a nozzle configured to spray a cleaning fluid toward a portion of the outer surface of the observation window 12. A liquid such as water or the like, or a gas such as carbon dioxide or the like may be used as the cleaning fluid. In the embodiment, water is used as the cleaning fluid. Hereinafter, a case in which cleaning water W is used as the cleaning fluid will be described.

The injection nozzle 31 is attached to the distal end of the cleaning pipe 32.

The cleaning pipe 32 is a pipe configured to supply the cleaning water W to the injection nozzle 31. For example, a pipe formed of a hard material such as a resin, a metal, or the like, may be employed as the cleaning pipe 32. A flexible water supply hose 37 is connected to the proximal end of the cleaning pipe 32. The water supply hose 37 is connected to a water supply section 110 disposed at the proximal end side of the insertion unit 2. The water supply section 110 supplies the cleaning water W to the water supply hose 37 under control of a control unit 110 or button manipulation or the like by the operator.

The cleaning pipe 32 of the cleaning unit 30 is accommodated in a cleaning unit receiving chamber 14. The cleaning unit receiving chamber 14 is formed at the distal end wall 11 of the distal end housing 10. The cleaning unit receiving chamber 14 is formed adjacent to the observation window 12. The cleaning unit receiving chamber 14 is formed so as to have an elliptical cross section such that a long diameter direction thereof is along a direction parallel to the long diameter direction of the observation window 12 when seen from the distal end surface 11a of the distal end wall 11. The cleaning unit receiving chamber 14 passes parallel to the axial direction of the distal end housing 10 from the distal end surface 11 a of the distal end wall 11 of the distal end housing 10 to the housing space 16.

A proximal end of the cleaning unit receiving chamber 14 is in communication with the housing space 16. A partition wall 33 is provided at a boundary portion between the cleaning unit receiving chamber 14 and the housing space 16. The partition wall 33 partitions the inside and the outside of the distal end housing 10. The partition wall 33 is provided to prevent the fluid or the solid matter of the outside from being introduced into the housing space 16 through the cleaning unit receiving chamber 14 and to enable movement of the cleaning pipe 32 in the cleaning unit receiving chamber 14. For example, the partition wall 33 is formed of a soft material. The cleaning pipe 32 of the cleaning unit 30 is provided to pass through the partition wall 33 formed of the soft material. That is, in the embodiment, since the partition wall 33 is bent when the cleaning pipe 32 is integrally pivoted with the pulley 35, the cleaning pipe 32 is pivoted while maintaining a water-tight state.

A distal end of the cleaning pipe 32 protrudes outward from the cleaning unit receiving chamber 14. Accordingly, the injection nozzle 31 is turned along a movement track of the distal end of the cleaning pipe 32 at outside the cleaning unit receiving chamber 14. The injection nozzle 31 is provided to spray the cleaning water W toward the outer surface of the observation window 12 adjacent to the cleaning unit receiving chamber 14. An operation range in which the injection nozzle 31 is turned is set to spray the cleaning water W to a region of the cover 13 overlapping at least an imaging view field of the imaging part 20.

The region in which the injection nozzle 31 can spray the cleaning water W is set to a size such that the injection nozzle 31 can spray the cleaning water W to a portion of the observation window 12 in a state in which the injection nozzle 31 is stopped within the operation range of the injection nozzle 31.

In the embodiment, the cleaning pipe 32 of the cleaning unit 30 is fixed to the pulley 35 using a fixing tool 34. The cleaning pipe 32 and the injection nozzle 31 are pivoted about a rotational center of the pulley 35. Accordingly, the injection nozzle 31 is swung in directions of arrows B1 and B2 (see Fig. 1) in a surface along the long diameter direction of the observation window 12 such that the injection nozzle 31 can spray the cleaning water W to the entire observation window 12.

A position of the distal end of the cleaning pipe 32 has a certain fixed positional relation with respect to the imaging part 20. That is, the distal end of the cleaning pipe 32 is directed to the imaging part 20 such that the injection nozzle 31 can spray the cleaning water W to the observation window 12 as if the injection nozzle 31 can spray the cleaning water W to the imaging part 20. Accordingly, the injection nozzle 31 of the distal end of the cleaning pipe 32 is configured to be directed to a position of the outer surface of the observation window 12 corresponding to a center of the imaging view field of the imaging part 20 such that the cleaning pipe 32 can always spray the cleaning water W to a position in the imaging view field of the imaging part 20.

In the embodiment, as the imaging part 20 is pivoted, a range of an imaging view field imaged by the imaging part 20 through the observation window 12 is changed by pivoting the imaging part 20 in the long diameter direction of the observation window 12. That is, an imaging part moving unit M1 configured to change the range of the imaging view field comprises the pulley 35, the manipulation wire 60, the connecting shaft 50, the key 51, the support member 21 and the actuator 101. When the operator manually manipulates the manipulation wire 60, components from the pulley 35 to a member configured to input a manual manipulation force are included in the imaging part moving unit M1.

When the pulley 35 is rotated, the cleaning unit 30 is pivoted in the long diameter direction of the observation window 12, and a cleaning position on the observation window 12 by the cleaning unit 30 is changed. A cleaning position change unit N1 changes a cleaning position being cleaned by the cleaning unit 30. That is, the cleaning position change unit N1 is used in common with the imaging part moving unit M1 including the pulley 35. In this case, the pulley 35 to which both of the cleaning unit 30 and the imaging part 20 are fixed corresponds to a synchronizing unit R1 configured to integrally move the cleaning unit 30 and the imaging part 20. The synchronizing unit R1 is a unit configured to synchronize a change operation for changing the range of the imaging view field of the imaging part 20 of the imaging part moving unit M1 and a change operation of the cleaning position of the cleaning unit 30 by the cleaning position change unit N1.

In the endoscope 1A according to the embodiment, the cleaning unit 30 is directly fixed to the pulley 35 using the fixing tool 34. A position of the injection nozzle 31 of the cleaning unit 30 coincides with a position of a center of the range of the imaging view field range of the imaging part 20 in a rotational direction of the pulley 35. Accordingly, in the endoscope 1A, as the pulley 35 is rotated, the cleaning position of the observation window 12 by the cleaning unit 30 is changed to a position where the observation window 12 includes an extended direction of the imaging view field of the imaging part 20, in accordance with a movement of the imaging part. That is, the cleaning position of the observation window 12 by the cleaning unit 30 is changed in accordance with the movement of the imaging part 20 such that the cleaning unit 30 cleans the observation window 20 positioned in front of the imaging view field of the imaging part 20.

In the endoscope 1A according to the embodiment, the cleaning unit 30 and the imaging part 20 are connected to be constantly pivoted in the same direction when the pulley 35 is rotated. Accordingly, the fixing tool 34 in which the cleaning unit 30 is directly fixed to the pulley 35 corresponds to a power transmission unit T1.

The endoscope 1A includes the control unit 100. The control unit 100 controls the actuator 101 for moving the imaging part 20 and the cleaning unit 30. The control unit 100 also controls a water supply operation of the water supply section 110 conveying the cleaning water W to the cleaning pipe 32 of the cleaning unit 30. The water supply section 110 includes a pump for pumping the cleaning water W, a valve for controlling supply and stoppage of the cleaning water W, or the like.

Next, a configuration of the control unit 100 provided at the endoscope 1A and an operation of the endoscope 1A according to the embodiment will be described with reference to Figs. 1 to 3.

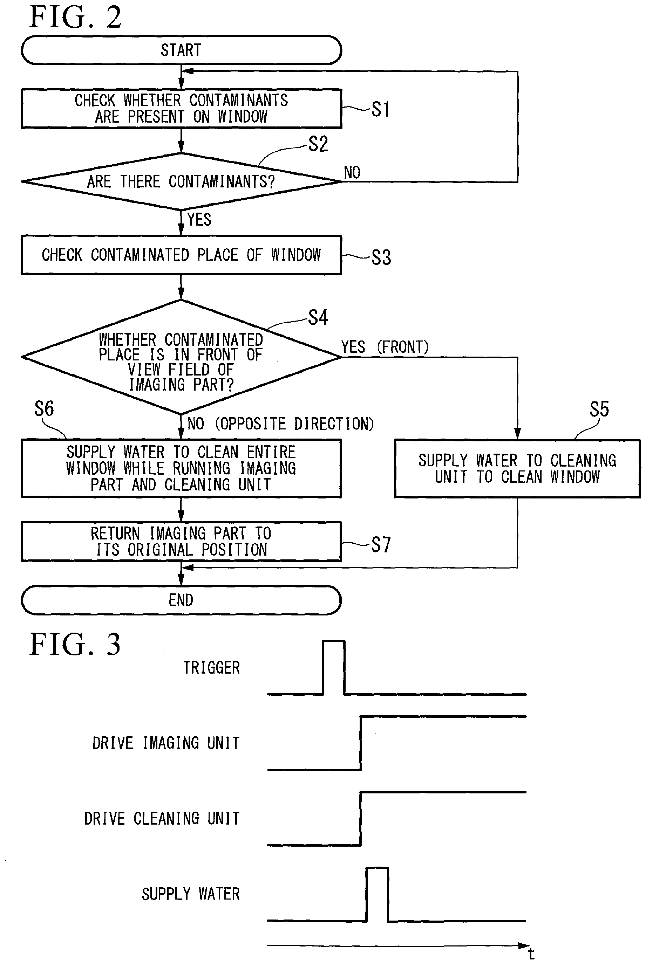

Fig. 2 is a flowchart showing a cleaning control flow performed in the endoscope 1A. Fig. 3 is a timing chart of a cleaning operation performed in the endoscope 1 A.

First, the operator of the endoscope 1A inserts a distal end side of the insertion unit 2 of the endoscope 1A into the body. The imaging part 20 is provided at the distal end housing 10 of the distal end of the insertion unit 2 inserted into the body. Accordingly, the imaging part 20 images an imaging target outside the distal end housing 10 through the observation window 12. The obtained image, which is image data, is transferred to an image control unit (not shown) outside the endoscope 1 A via a signal line (not shown) inserted into the insertion unit 2 and is displayed as an image on a display device (not shown).

The operator manipulates the manipulation unit provided at hand according to necessity while observing the image displayed on the display device. For example, the operator inputs a command signal that changes the range of the imaging view field of the imaging part 20 to the control unit 100. When the command signal that changes the range of the imaging view field of the imaging part 20 is input to the control unit 100, the control unit 100 outputs a driving signal to the actuator 101 to drive the actuator 101. When the actuator 101 is driven, the manipulation wire 60 is pushed and pulled. When the manipulation wire 60 is pushed and pulled, the pulley 35 is rotated. When the pulley 35 is rotated, the imaging part 20 and the cleaning unit 30 are integrally pivoted. That is, the imaging part 20 and the cleaning unit 30 are operated concurrently based on the control by the control unit 100.

When the imaging part 20 is pivoted, the range of the imaging view field of the imaging part 20 is changed. When the cleaning unit 30 is pivoted, the cleaning position by the cleaning unit 30 is changed. The control unit 100 includes a function of determining whether the cleaning is needed or not. For example, the control unit 100 may have a function of determining whether the observation window 12 is contaminated or not, or whether a contamination position of the observation window 12 is in front of the imaging view field direction of the imaging part or not.

When the cleaning is needed, the control unit 100 outputs a driving signal to the water supply section 110, and pumps the cleaning water W to the cleaning unit 30. When the cleaning water W is pumped to the cleaning unit 30, the injection nozzle 31 sprays the cleaning water W toward the outer surface of the observation window 12. Accordingly, contaminants on the observation window 12 are removed at a part in which the cleaning water W is sprayed.

Next, an example of a flow when the control unit 100 automatically controls the cleaning will be described with reference to the flowchart of Fig. 2.

When the operator inserts the distal end side of the insertion unit 2 of the endoscope 1A into the body and observes the inside of the body, contaminants are stuck to the observation window 12 of the endoscope distal end section and thus the imaging by the imaging part 20 may not be easily performed. As measures for above-mentioned circumstances, a program of the cleaning control is performed.

When the program of the cleaning control is started, first, in step S1, a checking operation of presence of the contaminants on the observation window 12 is performed. The checking of the presence of the contaminants on the observation window 12 is performed by, for example, a known method of processing an image displayed on the display device.

After step S1, step S2 is performed. Step S2 is a step of determining whether the contaminants are present using information obtained as a result of the checking operation in step S1. In step S2, a determination whether the contaminants are present or not is performed. In step 2, when determined that no contaminants are present, the flow returns to step S1, and the checking operation of the presence of the contaminants continues. In step 2, when determined that the contaminants are present, step S3 is performed.

In step S3, the checking operation of the contaminated place of the observation window 12 is performed. The checking of the contaminated place is also performed by, for example, a known method of processing the image displayed on the display device.

After step S3, step S4 is performed. In step S4, a contaminated place on the observation window 12 is determined. In the embodiment, a process is branched based on the contaminated place which is divided into two regions, i.e., a front surface in the imaging view field direction of the imaging part 20 and an outside in the imaging view field direction of the imaging part 20 (a side to which a center of the imaging view field of the imaging part 20 is not directed).

When the contaminated place is determined to be a position at the front surface in the imaging view field direction of the imaging part 20, step S5 for removing the contaminants attached to the front surface in the imaging view field direction of the imaging device 20 is performed. When the contaminated place is determined to be a position in the outside direction of the front surface in the imaging view field direction of the imaging part 20, step S6 for removing the contaminants fouled to the outside of the front surface in the imaging view field direction is performed.

When the imaging view field direction of the imaging part 20 is deviated to one side, the imaging view field direction of the imaging part is referred to as a side to which the imaging part 20 is deviated. In this case, the opposite direction of the front surface of the imaging view field direction of the imaging part is referred to as an opposite side of the side to which the imaging view field direction of the imaging part 20 is deviated.

In step S5, the supply of the cleaning water to the cleaning unit 30 is performed, and the cleaning of the observation window 12 is performed. At that time, since a direction of the imaging part 20 and a direction of the cleaning unit 30 are set to be same direction, the observation window 12 is cleaned at a position including an extended imaging view field direction of the imaging part 20. After the cleaning is performed in step S5, the cleaning processing is terminated, and the method is started again.

In step S6, an operation of supplying water and cleaning the entire window while moving the imaging part 20 and the cleaning unit 30 is performed. Here, step S6 is terminated, and step S7 is performed.

In step S7, an operation of returning the imaging part 20 to an original position thereof is performed. Here, the cleaning processing is terminated once, and the method is started again.

Next, the case in which the observation window 12 is cleaned in a timing at which the imaging view field direction of the imaging part 20 is changed will be described using a timing chart of Fig. 3.

The operation of changing the imaging view field direction of the imaging part 20 is likely to be performed when the operator wants to see the imaging target in the changed imaging view field. Therefore, the observation window 12 in the changed imaging view field may be in a cleaned state. In the embodiment, when a trigger signal serving as a command for changing the imaging view field is input into the control unit 100, the control unit 100 controls the imaging part 20 and the cleaning unit 30 to pivot in conjunction with each other. After that, continuously, the control unit 100 controls the injection nozzle 31 to spray the cleaning water W. That is, in the embodiment, the pivot operation of the imaging part 20 and the cleaning unit 30 is synchronized with a spraying operation of the cleaning water W.

Accordingly, when movement of the imaging part 20 for changing the imaging view field is terminated, the cleaning of the observation window 12 is also terminated, and the operator can perform appropriate treatment in the changed imaging view field.

In the timing chart shown in Fig. 3, in the timing from emission of a trigger signal to starting of water supply, a step of determining whether the contaminants are present and determining whether the water supply is needed may be included. In this case, the cleaning is not always performed whenever the imaging view field is changed, but the cleaning is performed only when the imaging view field is changed and the contaminants are present on the observation window 12 at the changed place.

In the endoscope 1A according to the embodiment, the spraying of the cleaning water W is performed in consideration of the imaging view field direction of the imaging part 20. That is, in the embodiment, the cleaning position by the cleaning unit 30 is changed in conjunction with a movement of the imaging part 20, and the cleaning unit 30 cleans the outer surface of the observation window 12 in the position including the imaging view field direction of the imaging part 20 to which the imaging part 20 is moved. Accordingly, the endoscope 1A according to the embodiment, in particular, can clean the observation window 12 at a position including the direction to be imaged by the imaging part 20 and the cleaning can be efficiently performed. In addition, the endoscope 1A according to the embodiment does not require a large cleaning unit that can clean the entire observation window 12 at once. In the endoscope 1A according to the embodiment, since the cleaning unit 30 does not spray the cleaning water W to a position of the observation window outside the imaging view field of the imaging part that need not be cleaned, waste of the cleaning water W can be reduced, and the cleaning can be efficiently performed.

In the first embodiment, the control unit 100 automatically determines the presence of contaminants or the contaminated place of the observation window 12. However, the operator can determine the presence of the contaminants or the contaminated place while observing the photographed image of the imaging part 20. In this case, the operator may directly input a cleaning command to the control unit 100.

### (Second embodiment)

Next, a second embodiment of the present invention will be described. In the embodiments described below, the same components as of the above-mentioned first embodiment are designated by the same reference numerals, and a description thereof will be omitted here.

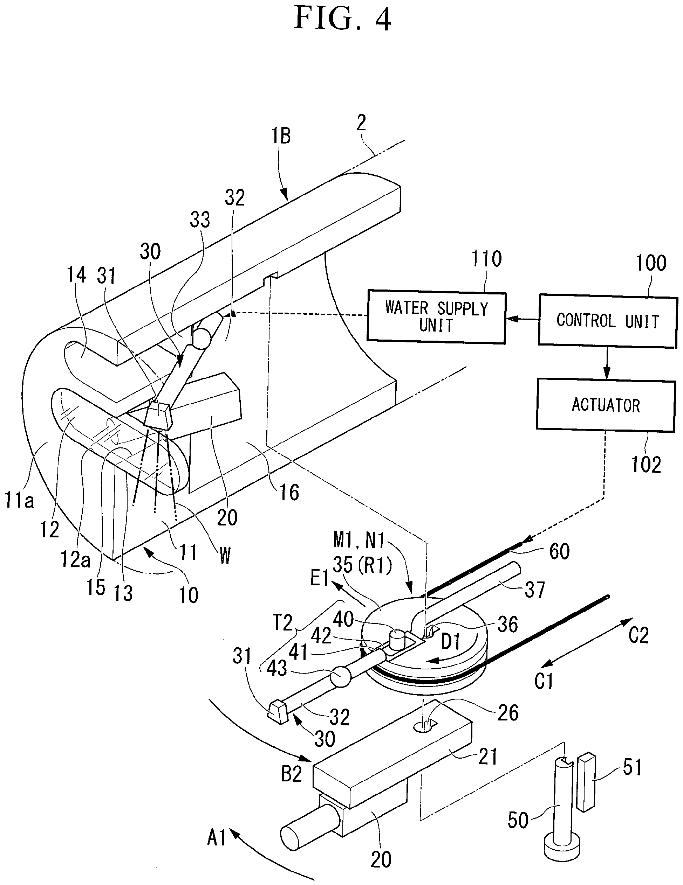

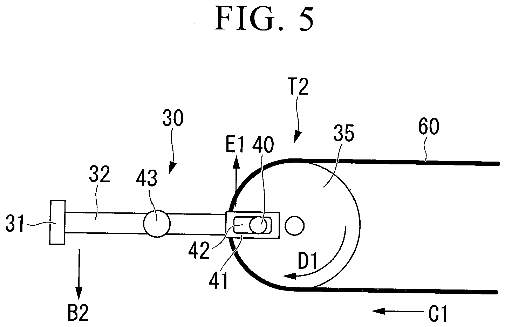

Fig. 4 is a perspective view schematically showing a configuration of a distal end of the endoscope according to the second embodiment. Fig. 5 is a plan view showing some of the components of the endoscope according to the embodiment.

As shown in Figs. 4 and 5, in an endoscope 1B according to the embodiment, a structure attaching the cleaning pipe 32 of the cleaning unit 30 to the pulley 35 is different from that of the endoscope 1A according to the first embodiment. In the endoscope 1B, instead of the fixing tool 34 shown in Fig. 1, the cleaning pipe 32 is connected to the pulley 35 using a fixing pin 40, a slide member 41 having a long hole 42, and a pivot shaft 43.

The fixing pin 40 is fixed to a side surface along the rotary surface of the pulley 35. A central axis of the fixing pin 40 extends parallel to the rotary shaft of the pulley 35.

The slide member 41 having the long hole 42 is attached to the proximal end section of the cleaning pipe 32. The long hole 42 is formed such that a direction of long diameter of the long hole 42 is directed to a central axial direction (a longitudinal direction) of the cleaning pipe 32.

The fixing pin 40 is slidably inserted into the long hole 42 of the slide member 41 to be engaged with the slide member 41.

The pivot shaft 43 is disposed at an intermediate portion between the distal end section and the proximal end section in the longitudinal direction of the cleaning pipe 32. The cleaning pipe 32 is swingably held by the pivot shaft 43 in the cleaning unit receiving chamber 14. Accordingly, the cleaning pipe 32 is pivotable about the pivot shaft 43 serving as a pivotal center in a surface parallel to the rotary shaft of the pulley 35.

That is, in the embodiment, the cleaning pipe 32 is configured to pivot about the pivot shaft 43 along the long diameter direction of the observation window 12 in accordance with pivoting of the pulley 35. Specifically, since the cleaning pipe 32 swings about the pivot shaft 43 in a seesaw manner, the distal end side of the cleaning pipe 32 is configured to swing in an opposite direction of the proximal end side of the cleaning pipe 32. In the embodiment, a length from the pivot shaft 43 to the injection nozzle 31 is set to be equal to a length from the pivot shaft 43 to the fixing pin 40. Accordingly, a displacement amount of the fixing pin 40 and a displacement amount of the injection nozzle 31 when the pulley 35 is rotated are equal to each other.

For example, when the pulley 35 is rotated about the rotary shaft in one direction along the rotary surface (a direction of an arrow D1 in Fig. 4) and the imaging part 20 is pivoted in the same direction of the arrow A1 as the pulley 35, the fixing pin 40 pushes the slide member 41. When the slide member 41 is pushed by the fixing pin 40, the slide member 41 is pivoted in a direction of an arrow E1 shown in Fig. 4 using the pivot shaft 43 as a support point. When the slide member 41 is pivoted in the direction of the arrow E1, the distal end side of the cleaning pipe 32 is pivoted in the direction of the arrow B2 opposite to the imaging part 20.

The cleaning position change unit N1 can change the cleaning position by the cleaning unit 30. In the embodiment, as the pulley 35 is rotated, the cleaning unit 30 is pivoted along the long diameter direction of the observation window 12, and the cleaning position of the observation window 12 by the cleaning unit 30 is changed. That is, the cleaning position change unit N1 is used in common with the imaging part moving unit M1. The pulley 35 is connected to both of the cleaning unit 30 and the imaging part 20. In the embodiment, the pulley 35 corresponds to the synchronizing unit R1 configured to synchronize the cleaning unit 30 with the imaging part 20.

In the endoscope 1B according to the embodiment, the cleaning unit 30 is connected to the pulley 35 using the fixing pin 40, the slide member 41 and the pivot shaft 43. That is, the fixing pin 40, the slide member 41 and the pivot shaft 43 that connect the cleaning unit 30 to the pulley 35 correspond to a power transmission unit T2. The power transmission unit T2 can change the cleaning position of the observation window 12 to a position to which includes a different direction from the imaging view field direction of the imaging part 20 which is moved by the imaging part moving unit M1.

Next, an action of the endoscope 1B according to the embodiment will be described with reference to Figs. 4 to 7.

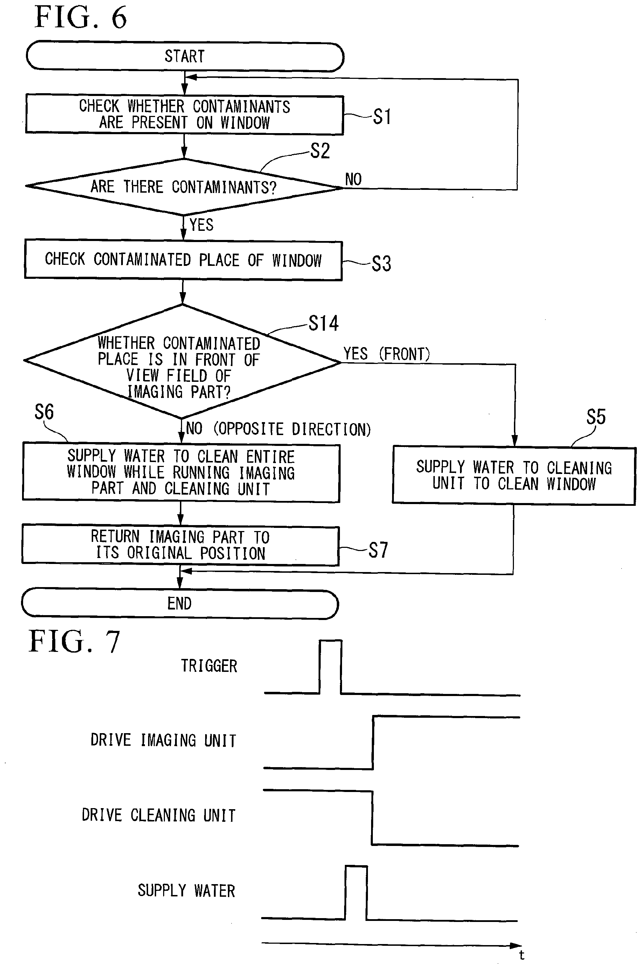

Fig. 6 is a flowchart showing a cleaning control flow performed in the endoscope 1B. Fig. 7 is a timing chart of a cleaning operation performed in the endoscope 1B.

A flow of the case in which the control unit 100 automatically performs the cleaning control will be described with reference to the flowchart of Fig. 6.

When a program of the cleaning control is started, step S1 to step S3 are performed in the same way as in the first embodiment.

In the embodiment, instead of step S4 described in the first embodiment, step S 14 in which processing contents are different is performed.

In step S14, the contaminated place is determined like step S4 in the first embodiment. Then, in step S 14, when the contaminated place is determined to be a position in the outside direction of the front surface in the imaging direction by the imaging part 20, step S5 is performed. In addition, when in step S14, the contaminated place is determined to be a position at the front surface in the imaging direction by the imaging part 20, step S6 is performed.

In step S5, like the first embodiment, water supply to the cleaning unit 30 is performed and cleaning of the observation window 12 is performed while maintaining a state in which the imaging part 20 is not moved. At that time, since the cleaning unit 30 is disposed at a different direction of a direction of the imaging part 20, the contaminants disposed at a different side of the imaging view field direction of the imaging part 20 are removed.

After the cleaning is performed in step S5, the cleaning processing is terminated once, and the method is restarted.

In step S6, an operation of supplying water and cleaning the entire window while moving the imaging part 20 and the cleaning unit 30 is performed. The embodiment is distinguished from the endoscope 1A according to the above-mentioned first embodiment in that a moving direction of the imaging part 20 is opposite to a moving direction of the cleaning unit 30. In the embodiment, step S6 is a step of spraying the cleaning water W while moving the injection nozzle 31 disposed at an opposite side of the imaging view field direction to the front surface in the direction of the imaging view field in step S14. At that time, the imaging part 20 is moved once from the front surface in the imaging view field direction to the opposite direction.

Here, step S6 is terminated, and step S7 is performed.

In step S7, an operation of returning the imaging part 20 to an original position thereof is performed. Here, the cleaning processing is terminated once, and the method is restarted. That is, in the embodiment, the imaging view field of the imaging part 20 is moved once to the different side when the front surface in the imaging view field direction is cleaned, and the imaging view field direction of the imaging part 20 is immediately returned to the original direction thereof after completion of the cleaning.

Next, the case in which the observation window 12 is cleaned at the timing at which the imaging view field direction of the imaging part 20 is changed will be described with reference to the timing chart of Fig. 7. Like the first embodiment, since the operation of changing the imaging view field direction of the imaging part 20 is likely to be performed when the operator wants to observe the imaging target in the changed imaging view field. Therefore, the observation window 12 in the changed imaging view field may be in a cleaned state. In the embodiment, when the trigger signal serving as a command for changing the imaging view field is input to the control unit 100, the control unit 100 controls the cleaning unit 30 to spray the cleaning water W. After that, the imaging part 20 and the cleaning unit 30 are pivoted in interrelation. That is, in the embodiment, the pivotal operation of the imaging part 20 and the cleaning unit 30 is synchronized with the spraying operation of the cleaning water W.

Accordingly, since the cleaning of the observation window 12 at the moved place is also terminated before movement of the imaging part 20 for changing the imaging view field direction is started, the operator can immediately start appropriate treatment in the changed imaging view field.

### (Third embodiment)

Next, a third embodiment of the present invention will be described.

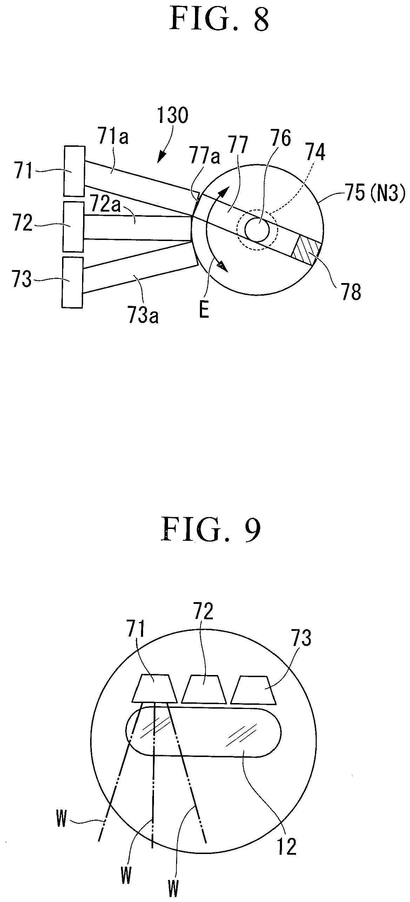

Figs. 8 and 9 are views showing configurations of a cleaning unit and a cleaning position change unit included in an endoscope according to the third embodiment of the present invention, Fig. 8 is a plan view and Fig. 9 is a front view.

In the first embodiment and the second embodiment, the case in which change of the cleaning position by the cleaning unit 30 is performed by moving the injection nozzle 31 of the cleaning unit 30 with respect to the observation window 12 has been described. On the other hand, in the embodiment, the case in which the change of the cleaning position by the cleaning unit is performed as an injection nozzle of the cleaning unit is in a fixed state and the injection nozzle is selected from the plurality of injection nozzles will be described.

As shown in Figs. 8 and 9, a cleaning unit 130 of the embodiment has a plurality of injection nozzles 71, 72 and 73 configured to clean a plurality of regions of the outer surface of the observation window 12. The plurality of injection nozzles 71, 72 and 73 are regional cleaning units corresponding to a plurality of regions of the outer surface of the observation window 12. The injection nozzles 71, 72 and 73 are arranged in the long diameter direction of the observation window 12.

The injection nozzle 71 of which disposed at a first end in a direction in which the injection nozzles 71, 72 and 73 are arranged is configured to clean a region adjacent to a first end in the long diameter direction of the observation window 12. The injection nozzle 72 in the middle of the arrangement direction is configured to clean a middle region in the long diameter direction of the observation window 12. The injection nozzle 73 of a second end in the arrangement direction is configured to clean a region adjacent to a second end in the long diameter direction of the observation window 12.

A flow path switching member 75 having a columnar shape is assembled to the arrangement of the injection nozzles 71, 72 and 73. The flow path switching member 75 is a member configured to select which of the injection nozzles 71, 72 and 73 to supply water thereto. The flow path switching member 75 is rotatably supported by a rotary shaft 74 having a rotational axis parallel to a rotational axis of the pulley 35.

A switching flow path 77 which is performed water supply from the water supply hose 37 (see Fig. 1) via a passage 76 in the rotary shaft 74 is provided in the flow path switching member 75.. The switching flow path 77 is formed in the radial direction of the flow path switching member 75. The first end of the switching flow path 77 is closed by a stopper 78, and the second end is opened at an outer circumference of the flow path switching member 75.

Pipes 71a, 72a and 73a are provided at the injection nozzles 71, 72 and 73, respectively. Pipes 71a, 72a and 73a are slidable and hermetical communication with an opening 77a of the rotating flow path switching member 75. Accordingly, the flow path switching member 75 is configured to make the switching flow path 77 to communicate with only one of the injection nozzles 71, 72 and 73 according to rotation of the flow path switching member 75. That is, the cleaning position of the observation window 12 is changed according to which of the injection nozzles 71, 72 and 73 is in communication with the switching flow path 77. In the embodiment, the flow path switching member 75 corresponds to a cleaning position change unit N3.

The flow path switching member 75 is connected to the pulley 35 (see Fig. 1) via a power transmission unit (not shown). Since the rotational axis of the flow path switching member 75 is parallel to the rotational axis of the pulley 35, the connecting shaft 50 (see Fig. 1) may be directly used as the rotary shaft 74. In addition, a separate power transmission unit may be interposed between the pulley 35 and the flow path switching member 75. Since the flow path switching member 75 and the pulley 35 are connected to each other via the power transmission unit, the flow path switching member 75 is configured to be rotated in conjunction with the pulley 35. Accordingly, in the embodiment, the power transmission unit corresponds to a synchronizing unit.

In the endoscope according to the embodiment, as one of the injection nozzles 71, 72 and 73 arranged in the long diameter direction of the observation window 12 is selected, the cleaning position with respect to the observation window 12 is changed. Accordingly, a compact structure in which a member configured to switch a flow path with respect to the injection nozzles 71, 72 and 73 is provided is realized.

In the endoscope according to the embodiment, since the injection nozzles 71, 72 and 73 are fixed to the distal end housing 10, a water-tight state of the endoscope is more easily and securely realized than in the configurations shown in the above-mentioned embodiments.

### (Fourth embodiment)

Next, a fourth embodiment of the present invention will be described.

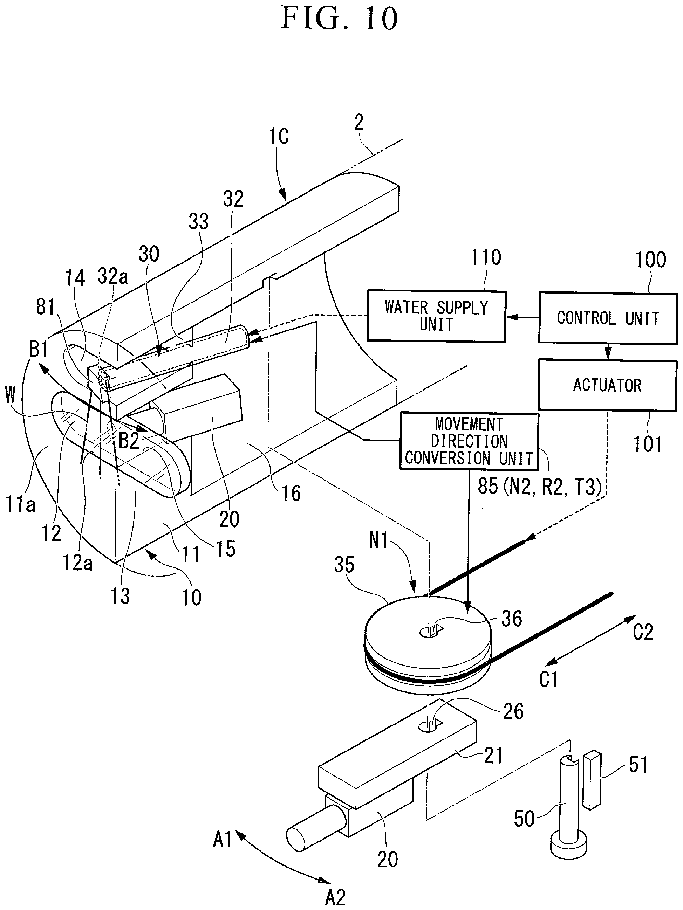

Fig. 10 is a perspective view schematically showing a configuration of a distal end of an endoscope according to the fourth embodiment of the present invention.

In the first embodiment and the second embodiment, the case in which the change of the cleaning position of the cleaning unit 30 is performed as the cleaning pipe 32 and the injection nozzle 31 of the cleaning unit 30 are moved with respect to the observation window 12 has been described. On the other hand, in an endoscope 1C according to the embodiment, as shown in Fig. 10, the cleaning pipe 32 of the cleaning unit 30 is in a fixed state, and a movable reflective plate 81 is provided at a position facing a distal end opening 32a of the cleaning pipe 32. In the endoscope 1C, as an orientation of the reflective plate 81 is varied, the change of the cleaning position of the cleaning unit 30 is performed.

The cleaning unit 30 of the endoscope 1C according to the embodiment has the cleaning pipe 32 and the movable reflective plate 81. The cleaning pipe 32 is fixed to the distal end housing 10. The reflective plate 81 is attached to the cleaning pipe 32. In the embodiment, unlike the first embodiment or the second embodiment, the injection nozzle 31 configured to directly spray the cleaning water toward the outer surface of the observation window 12 is not provided at the distal end of the cleaning pipe 32. Instead of this, the distal end opening 32a of the cleaning pipe 32 is directed toward the reflective plate 81.

The reflective plate 81 reflects the cleaning water W sprayed from the distal end opening 32a of the cleaning pipe 32 toward the outer surface of the observation window 12. The reflected cleaning water is configured to be sprayed to the outer surface of the observation window 12 to clean the observation window 12.

A rod 80 configured to change a direction of the reflective plate 81 is connected to the reflective plate 81. The rod 80 is connected to the pulley 35 via a movement direction conversion unit 85. The movement direction conversion unit 85 is a unit configured to convert rotation of the pulley 35 into an operation of changing the direction of the reflective plate 81 and to transmit the operation to the rod 80. The reflective plate 81 is configured such that a position to which the cleaning water W ejected from the distal end opening 32a of the cleaning pipe 32 is sprayed to the observation window 12 can be changed by adjusting the direction of the reflective plate 81 by the movement direction conversion unit 85 and the rod 80.

In the endoscope 1C according to the embodiment, a cleaning position change unit N2 configured to change a cleaning position of the cleaning unit 30 is comprised by the reflective plate 81, the rod 80, and the movement direction conversion unit 85 or the like. A synchronizing unit R2 and a power transmission unit T3 are constituted by the movement direction conversion unit 85.

In the endoscope 1C according to the embodiment, the same effects as of the first embodiment and the second embodiment are exhibited.

Further, according to the endoscope 1C according to the embodiment, the cleaning pipe 32 may not be moved, and a spray direction of the cleaning water can be changed by a simple mechanism. Accordingly, in the embodiment, a structure of the cleaning position change unit N2 can make compact.

### (Fifth embodiment)

Next, a fifth embodiment of the present invention will be described.

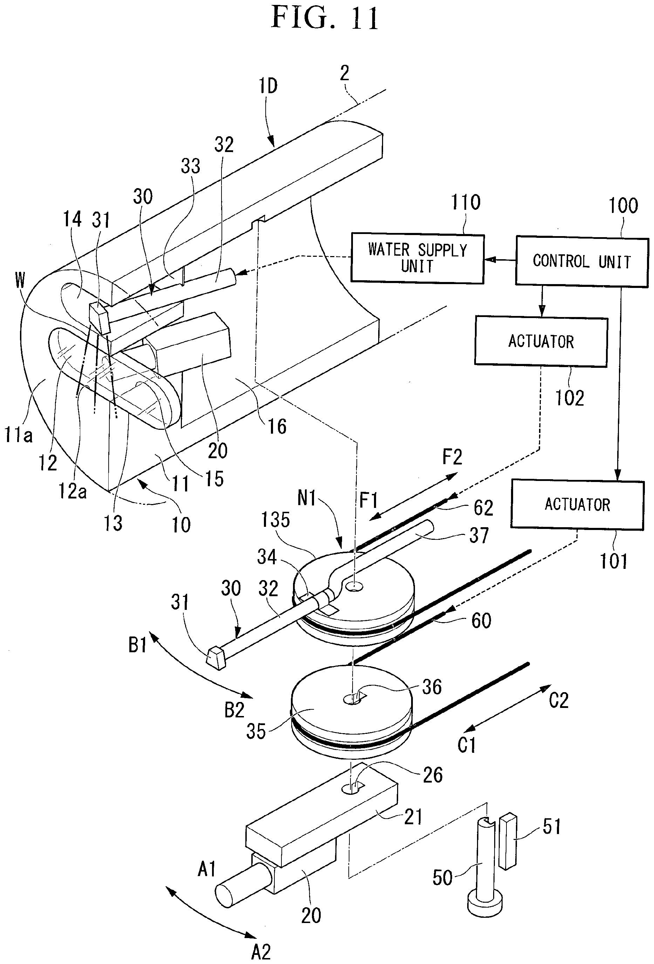

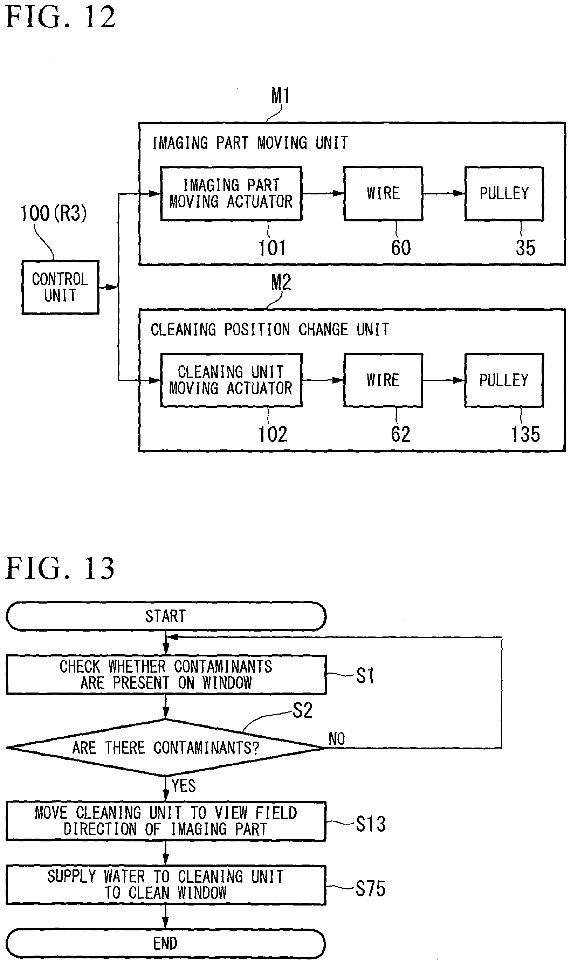

Fig. 11 is a perspective view schematically showing a configuration of a distal end of an endoscope according to the fifth embodiment of the present invention. Fig. 12 is a block diagram showing a configuration of a driving system of the endoscope.

In the first embodiment and the second embodiment, the configuration in which the imaging part moving unit M1 and the cleaning position change unit N1 are used in common in the same driving system was described. However, the driving systems of the imaging part moving unit M1 and the cleaning position change unit N1 may be separately configured.

As shown in Figs. 11 and 12, in an endoscope 1D according to the fifth embodiment, the imaging part moving unit M1 and the cleaning position change unit N1 are independently provided as separate driving systems.

The imaging part moving unit M1 has the actuator (the driving unit) 101, the manipulation wire 60, and the pulley 35. The cleaning position change unit N1 includes an actuator (a driving unit) 102 configured to move the cleaning unit 30, a manipulation wire 62, and a pulley 135.

The actuator 102, the manipulation wire 62 and the pulley 135 of the cleaning position change unit N1 are configured to rotate the pulley by pushing and pulling the wire, like the actuator 101, the manipulation wire 60 and the pulley 35 of the imaging part moving unit M1. That is, in the embodiment, the pulley 135 is rotated by pushing and pulling the manipulation wire 62 in directions of arrows F1 and F2.

The cleaning pipe 32 of the cleaning unit 30 is fixed to the pulley 135 of the cleaning position change unit N1 by using the fixing tool 34. Accordingly, the cleaning unit 30 is configured to be pivoted in the directions of the arrows B1 and B2 by rotating the pulley 135.

The control unit 100 includes a function of moving the actuator 101 of the imaging part moving unit M1 and the actuator 102 of the cleaning position change unit N1 so as to be synchronized with each other. That is, in the embodiment, the control unit 100 corresponds to a synchronizing unit R3.

Next, an action of the endoscope 1D according to the embodiment will be described with reference to Figs. 11 to 14.

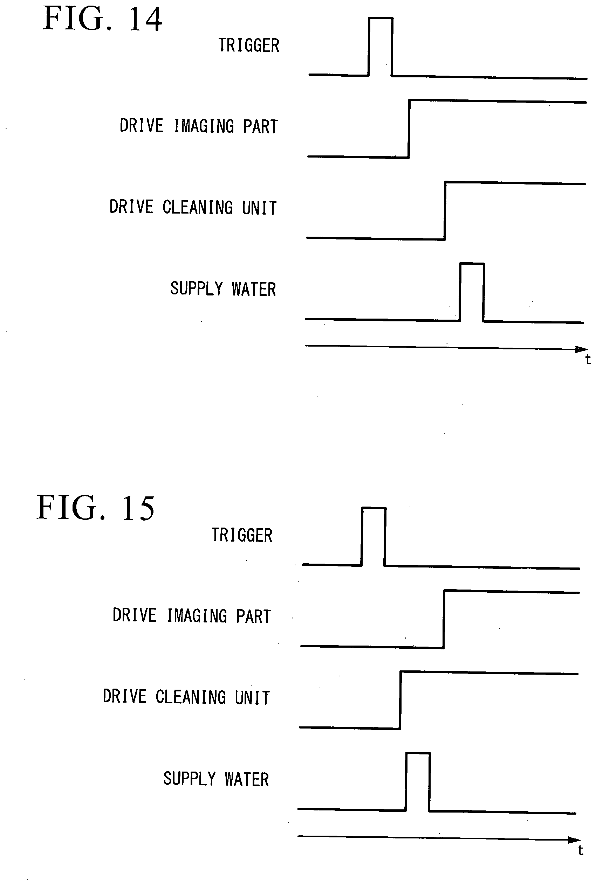

Fig. 13 is a flowchart showing a cleaning control flow performed in the endoscope 1D. Fig. 14 is a timing chart of a cleaning operation performed in the endoscope 1D.

A flow of the case in which the control unit 100 automatically performs the cleaning control will be described with reference to the flowchart of Fig. 13.

When a program of the cleaning control is started, like the first embodiment, a checking operation of presence of contaminants of the observation window 12 is performed in step S1, and the presence of the contaminants is determined in step S2.

When the contaminants are present, step S3 is performed after step S2. In step S3, the cleaning unit 30 follows the imaging part 20 to move in the imaging view field direction of the imaging part 20. Movement of the cleaning unit 30 may be performed to follow the imaging part 20 during movement of the imaging part 20, or may be performed after stoppage of movement of the imaging part 20.

After step S3, step S5 is performed. In step S5, water supply to the cleaning unit 30 is performed, and the outer surface of the observation window 12 at a position in the imaging view field direction of the imaging part 20 is cleaned.

In step S5, after the cleaning is performed, the cleaning processing is terminated once, and the method is restarted.

The timing of the cleaning operation according to the embodiment will be described using the timing chart of Fig. 14.

The control unit 100 performs control of the flowchart shown in Fig. 13 as a trigger signal is input. Then, when the cleaning is needed, the control unit 100 outputs a driving signal to the actuator 101 of the imaging part moving unit M1, the actuator 102 of the cleaning position change unit N1, and the water supply section 110. When the actuator 101 is operated, the imaging part 20 is pivoted. In accordance with a rotation of the imaging part 20, when the actuator 102 is operated, the cleaning unit 30 is pivoted. In addition, when the water supply section 110 is operated, the cleaning water is pumped to the cleaning unit 30 to perform the cleaning.

Although the imaging part 20 and the cleaning unit 30 can be independently moved, the imaging part 20 and the cleaning unit 30 are controlled to move in interrelation. In the timing chart of Fig. 14, the cleaning unit 30 is moved after moving the imaging part 20. After that, the water supply is performed. Accordingly, according to the endoscope 1D of the embodiment can effectively clean the contaminated place without changing the imaging view field of the imaging part 20.

The endoscope 1D according to the embodiment can independently move the imaging part 20 and the cleaning unit 30 at an arbitrary timing. However, operations of the imaging part 20 and the cleaning unit 30 are controlled in interrelation. Accordingly, the endoscope 1D according to the embodiment can variously combine the operations of the imaging part 20, the cleaning unit 30 and the water supply section 110.

Hereinafter, examples of operations that can be performed by the endoscope 1D will be described with reference to Figs. 15 to 19.

Figs. 15 to 19 are timing charts showing examples of operations of cleaning the observation window 12.

The timing chart of Fig. 15 is shown an example in which the cleaning unit 30 is first moved, and the imaging part 20 is moved after the water supply is performed. According to the operation example, an effect in which the imaging part 20 moving thereafter can be imaged through the portion of the observation window 12 from which the contaminants are removed is exhibited.

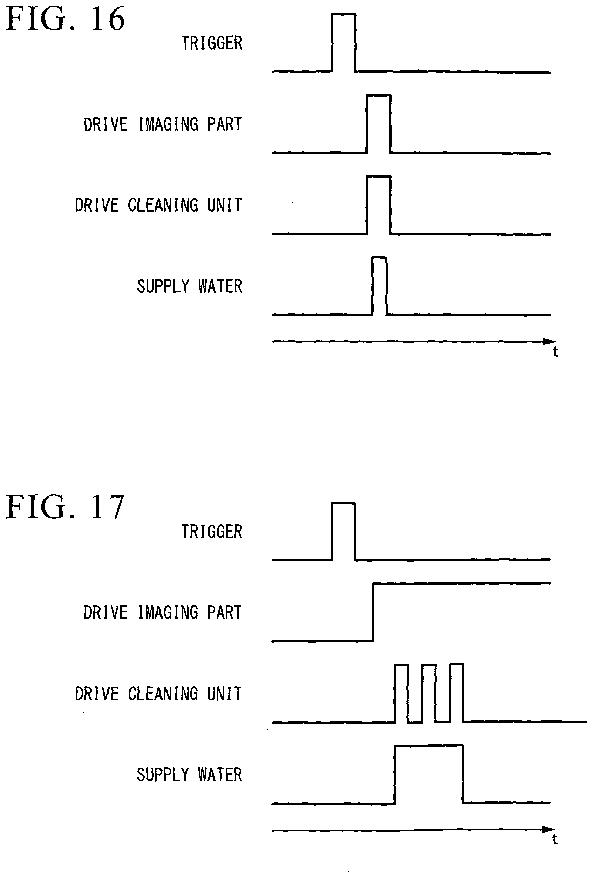

In the timing chart of Fig. 16, an example of the case in which the water supply is performed at the same timing as reciprocal movement of the imaging part 20 and reciprocal movement of the cleaning unit 30 is shown. In the operation example, when the observation window 12 in an observed imaging view field direction is contaminated, the imaging part 20 and the cleaning unit 30 are instantly and integrally moved to supply the water, and returned to their original positions after the cleaning.

The timing chart of Fig. 17 is shown an example of the case in which, after the imaging part 20 is moved, the cleaning unit 30 is frequently and repeatedly reciprocated, and the water supply is continuously performed therebetween. According to the operation example, an effect in which the entire surface of the observation window 12 is cleaned by the cleaning unit 30, which is frequently reciprocated, is exhibited.

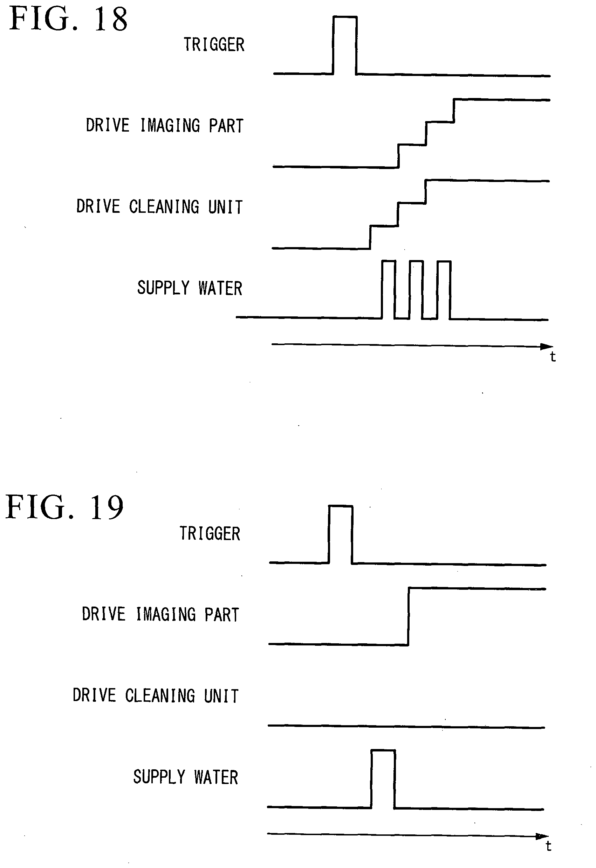

The timing chart of Fig. 18 is shown an example of the case in which "operations of moving the cleaning unit 30, supplying water and moving the imaging part 20" are repeated. In the operation example, the cleaning of the observation window 12 is performed by slightly moving the cleaning unit 30 while cleaning a moving front side of the imaging part 20.

The timing chart of Fig. 19 is shown an example of the case in which the water supply is performed just before movement of the imaging part 20, and the cleaning unit 30 is not moved. In the operation example, the water supply, i.e., the cleaning operation, is performed in conjunction with the moving operation of the imaging part 20. The water supply timing may be after the movement of the imaging part 20. In addition, the water supply timing may be in the middle of the movement of the imaging part 20.

In the case of the example shown in Fig. 19, a function of controlling the water supply section 110 in the control unit 100 corresponds to a synchronizing unit configured to synchronize a change operation of an imaging view field range with respect to the imaging part 20 of the imaging part moving unit M1 with a cleaning operation of the cleaning unit 30.

In the embodiment, while an example in which the cleaning unit 30 is comprised by the injection nozzle 31 and the cleaning pipe 32 has been described, instead of the example, the cleaning unit may be comprised by a wiper configured to clean the outer surface of the observation window 12.

Hereinabove, while the embodiments of the present invention have been described, the technical scope of the present invention is not limited to the embodiments, but combinations of the components of the embodiments, additions of various modifications to the components, and omissions of the components may be made without departing from the spirit of the present invention. The present invention is not limited to the above-mentioned description but is limited by only the scope of the accompanying claims.

### Industrial Applicability

According to the endoscope, it is possible to provide the endoscope capable of cleaning a position of the observation window in a direction which is to be imaged by the imaging part, and effectively performing the cleaning.

### Reference Signs List

- 1A, 1B, 1C, 1D:: endoscope
- 2:: insertion unit (endoscope insertion unit)
- 10:: distal end housing
- 12:: observation window
- 20:: imaging part
- M1:: imaging part moving unit
- 30:: cleaning unit
- R1, R2, R3:: synchronizing unit
- N1, N2, N3:: cleaning position change unit
- 71, 72, 73:: injection nozzle (regional cleaning unit)
- 101:: actuator (first driving unit)
- 102:: actuator (second driving unit)
- 31:: injection nozzle
- 100:: control unit
- 81:: reflective plate

## Claims

1. An endoscope comprising:
a distal end housing which is provided at a distal end of an endoscope insertion unit;
an observation window which is provided at the distal end housing and configured to be capable of observing an outside of the distal end housing from the inside of the distal end housing;
an imaging part which is provided inside the distal end housing and configured to image an imaging target which is positioned outside the distal end housing through the observation window;
an imaging part moving unit which is configured to move the imaging part with respect to the observation window and to change a range of an imaging view field of the imaging part; and a cleaning unit configured to clean an outer surface of the observation window; **characterized by** a synchronizing unit which is configured to synchronize a change operation of a range of an imaging view field of the imaging part by the imaging part moving unit with a cleaning operation by the cleaning unit.

2. The endoscope according to claim 1, further comprising
a cleaning position change unit which is configured to change a cleaning position on the observation window to be cleaned by the cleaning unit, wherein the synchronizing unit synchronizes the change operation of a range of the imaging view field of the imaging part by the imaging part moving unit with a change operation of the cleaning position by the cleaning position change unit.

3. The endoscope according to claim 2,
wherein the cleaning position change unit uses a driving force in common with the imaging part moving unit by being connected to the imaging part moving unit via a power transmission unit serving as the synchronizing unit.

4. The endoscope according to claim 3,
wherein the cleaning position change unit is connected to the imaging part moving unit via the power transmission unit such that the cleaning position is changed to a position of the observation window including an imaging view field direction of the imaging part which is moved by the imaging part moving unit.

5. The endoscope according to claim 3,
wherein the cleaning position change unit is able to change a cleaning position of the observation window to a position which is different from an imaging view field direction of the imaging part which is moved by the imaging part moving unit and the cleaning position change unit is connected to the imaging part moving unit via the power transmission unit.

6. The endoscope according to claim 2,
wherein the cleaning unit has a plurality of regional cleaning units configured to clean a plurality of regions of the outer surface of the observation window, and
the cleaning position change unit changes the cleaning position by selecting at least one regional cleaning unit from the plurality of regional cleaning units.

7. The endoscope according to claim 2,
wherein the imaging part moving unit has a first driving unit and the cleaning position change unit has a second driving unit, and
the synchronizing unit is a control unit which is configured to synchronize the first driving unit with the second driving unit.

8. The endoscope according to claim 1,
wherein the cleaning unit includes an injection nozzle configured to spray a cleaning fluid toward the outer surface of the observation window.

9. The endoscope according to claim 8,
wherein the synchronizing unit is a control unit configured to perform a spray control of the fluid from the injection nozzle so as to be synchronized with the change operation of a range of the imaging view field of the imaging part by the imaging part moving unit.

10. The endoscope according to claim 2,
wherein the cleaning unit includes an injection nozzle configured to spray a cleaning fluid toward the outer surface of the observation window, and
the cleaning position change unit changes a cleaning position by moving a direction of the injection nozzle.

11. The endoscope according to claim 2, wherein the cleaning unit includes an injection nozzle configured to spray a cleaning fluid toward the outer surface of the observation window, and
the cleaning position change unit has a reflective plate configured to reflect the cleaning fluid which is sprayed from the injection nozzle toward the outer surface of the observation window, and changes a cleaning position using the cleaning fluid by moving a direction of the reflective plate.

## Patentansprüche

1. Endoskop, umfassend:
ein distales Endgehäuse, das an einem distalen Ende einer Endoskopeinführungseinheit vorgesehen ist;
ein Betrachtungsfenster, das an dem distalen Endgehäuse vorgesehen ist und dazu konfiguriert ist, eine Außenseite des distalen Endgehäuses von der Innenseite des distalen Endgehäuses betrachten zu können;
einen Abbildungsteil, der im Inneren des distalen Endgehäuses vorgesehen ist und dazu konfiguriert ist, ein Abbildungsziel, das außerhalb des distalen Endgehäuses positioniert ist, durch das Betrachtungsfenster abzubilden;
eine Abbildungsteilbewegungseinheit, die dazu konfiguriert ist, den Abbildungsteil in Bezug auf das Betrachtungsfenster zu bewegen und einen Bereich eines Abbildungssichtfelds des Abbildungsteils zu ändern; und
eine Reinigungseinheit, die dazu konfiguriert ist, eine Außenfläche des Betrachtungsfensters zu reinigen;
**gekennzeichnet durch**
eine Synchronisierungseinheit, die dazu konfiguriert ist, einen Änderungsvorgang eines Bereichs eines Abbildungssichtfelds des Abbildungsteils durch die Abbildungsteilbewegungseinheit mit einem Reinigungsvorgang durch die Reinigungseinheit zu synchronisieren.

2. Endoskop nach Anspruch 1, weiterhin umfassend:
eine Reinigungspositionsänderungseinheit, die dazu konfiguriert ist, eine Reinigungsposition auf dem Betrachtungsfenster, die durch die Reinigungseinheit gereinigt werden soll, zu ändern,
wobei die Synchronisierungseinheit den Änderungsvorgang eines Bereichs des Abbildungssichtfelds des Abbildungsteils durch die Abbildungsteilbewegungseinheit mit einem Änderungsvorgang der Reinigungsposition durch die Reinigungspositionsänderungseinheit synchronisiert.

3. Endoskop nach Anspruch 2,
wobei die Reinigungspositionsänderungseinheit eine Antriebskraft gemeinsam mit der Abbildungsteilbewegungseinheit verwendet, indem sie mit der Abbildungsteilbewegungseinheit mittels einer Leistungsübertragungseinheit verbunden ist, die als die Synchronisierungseinheit dient.

4. Endoskop nach Anspruch 3,
wobei die Reinigungspositionsänderungseinheit mit der Abbildungsteilbewegungseinheit mittels der Leistungsübertragungseinheit verbunden ist, so dass die Reinigungsposition zu einer Position des Betrachtungsfensters geändert wird, die eine Abbildungssichtfeldrichtung des Abbildungsteils umfasst, der durch die Abbildungsteilbewegungseinheit bewegt wird.

5. Endoskop nach Anspruch 3,
wobei die Reinigungspositionsänderungseinheit eine Reinigungsposition des Betrachtungsfensters zu einer Position ändern kann, die sich von einer Abbildungssichtfeldrichtung des Abbildungsteils unterscheidet, der durch die Abbildungsteilbewegungseinheit bewegt wird, und die Reinigungspositionsänderungseinheit mit der Abbildungsteilbewegungseinheit mittels der Leistungsübertragungseinheit verbunden ist.

6. Endoskop nach Anspruch 2,
wobei die Reinigungseinheit mehrere regionale Reinigungseinheiten aufweist, die dazu konfiguriert sind, mehrere Regionen der Außenfläche des Betrachtungsfensters zu reinigen, und
die Reinigungspositionsänderungseinheit die Reinigungsposition durch Auswählen mindestens einer regionalen Reinigungseinheit aus den mehreren regionalen Reinigungseinheiten ändert.

7. Endoskop nach Anspruch 2,
wobei die Abbildungsteilbewegungseinheit eine erste Antriebseinheit aufweist und die Reinigungspositionsänderungseinheit eine zweite Antriebseinheit aufweist und
die Synchronisierungseinheit eine Steuereinheit ist, die dazu konfiguriert ist, die erste Antriebseinheit mit der zweiten Antriebseinheit zu synchronisieren.

8. Endoskop nach Anspruch 1,
wobei die Reinigungseinheit eine Einspritzdüse umfasst, die dazu konfiguriert ist, eine Reinigungsflüssigkeit in Richtung der Außenfläche des Betrachtungsfensters zu sprühen.

9. Endoskop nach Anspruch 8,
wobei die Synchronisierungseinheit eine Steuereinheit ist, die dazu konfiguriert ist, eine Sprühsteuerung der Flüssigkeit aus der Einspritzdüse durchzuführen, so dass sie mit dem Änderungsvorgang eines Bereichs des Abbildungssichtfelds des Abbildungsteils durch die Abbildungsteilbewegungseinheit synchronisiert ist.

10. Endoskop nach Anspruch 2,
wobei die Reinigungseinheit eine Einspritzdüse umfasst, die dazu konfiguriert ist, eine Reinigungsflüssigkeit in Richtung der Außenfläche des Betrachtungsfensters zu sprühen, und
die Reinigungspositionsänderungseinheit eine Reinigungsposition durch Bewegen einer Richtung der Einspritzdüse ändert.

11. Endoskop nach Anspruch 2,
wobei die Reinigungseinheit eine Einspritzdüse umfasst, die dazu konfiguriert ist, eine Reinigungsflüssigkeit in Richtung der Außenfläche des Betrachtungsfensters zu sprühen, und
die Reinigungspositionsänderungseinheit eine reflektierende Platte aufweist, die dazu konfiguriert ist, die Reinigungsflüssigkeit zu reflektieren, die aus der Einspritzdüse in Richtung der Außenfläche des Betrachtungsfensters gesprüht wird, und eine Reinigungsposition unter Verwendung der Reinigungsflüssigkeit durch Bewegen einer Richtung der reflektierenden Platte ändert.

## Revendications

1. Endoscope comprenant :
un logement d'extrémité distale qui est disposé à une extrémité distale d'une unité d'insertion d'endoscope ;
une fenêtre d'observation qui est disposée au niveau du logement d'extrémité distale et configurée pour être capable d'observer un extérieur du logement d'extrémité distale depuis l'intérieur du logement d'extrémité distale ;
une partie d'imagerie qui est disposée à l'intérieur du logement d'extrémité distale et configurée pour imager une cible d'imagerie qui est positionnée à l'extérieur du logement d'extrémité distale à travers la fenêtre d'observation ;
une unité de déplacement de partie d'imagerie qui est configurée pour déplacer la partie d'imagerie par rapport à la fenêtre d'observation et pour changer une portée d'un champ de visualisation d'imagerie de la partie d'imagerie ; et
une unité de nettoyage configurée pour nettoyer une surface externe de la fenêtre d'observation ;
**caractérisé par**
une unité de synchronisation qui est configurée pour synchroniser une opération de changement d'une portée d'un champ de visualisation d'imagerie de la partie d'imagerie par l'unité de déplacement de partie d'imagerie avec une opération de nettoyage par l'unité de nettoyage.

2. Endoscope selon la revendication 1, comprenant en outre :
une unité de changement de position de nettoyage qui est configurée pour changer une position de nettoyage sur la fenêtre d'observation à nettoyer par l'unité de nettoyage,
dans lequel l'unité de synchronisation synchronise l'opération de changement d'une portée du champ de visualisation d'imagerie de la partie d'imagerie par l'unité de déplacement de partie d'imagerie avec une opération de changement de la position de nettoyage par l'unité de changement de position de nettoyage.

3. Endoscope selon la revendication 2,
dans lequel l'unité de changement de position de nettoyage utilise une force d'entraînement en commun avec l'unité de déplacement de partie d'imagerie en étant reliée à l'unité de déplacement de partie d'imagerie par l'intermédiaire d'une unité de transmission de puissance servant en tant qu'unité de synchronisation.

4. Endoscope selon la revendication 3,
dans lequel l'unité de changement de position de nettoyage est reliée à l'unité de déplacement de partie d'imagerie par l'intermédiaire de l'unité de transmission de puissance de telle sorte que la position de nettoyage est changée à une position de la fenêtre d'observation comprenant une direction de champ de visualisation d'imagerie de la partie d'imagerie qui est déplacée par l'unité de déplacement de partie d'imagerie.

5. Endoscope selon la revendication 3,
dans lequel l'unité de changement de position de nettoyage est apte à changer une position de nettoyage de la fenêtre d'observation à une position qui est différente d'une direction de champ de visualisation d'imagerie de la partie d'imagerie qui est déplacée par l'unité de déplacement de partie d'imagerie et l'unité de changement de position de nettoyage est reliée à l'unité de déplacement de partie d'imagerie par l'intermédiaire de l'unité de transmission de puissance.

6. Endoscope selon la revendication 2,
dans lequel l'unité de nettoyage présente une pluralité d'unités de nettoyage régionales configurées pour nettoyer une pluralité de régions de la surface externe de la fenêtre d'observation, et
l'unité de changement de position de nettoyage change la position de nettoyage par sélection d'au moins une unité de nettoyage régionale parmi la pluralité d'unités de nettoyage régionales.

7. Endoscope selon la revendication 2,
dans lequel l'unité de déplacement de partie d'imagerie présente une première unité d'entraînement et l'unité de changement de position de nettoyage présente une seconde unité d'entraînement, et
l'unité de synchronisation est une unité de commande qui est configurée pour synchroniser la première unité d'entraînement avec la seconde unité d'entraînement.

8. Endoscope selon la revendication 1,
dans lequel l'unité de nettoyage comprend une buse d'injection configurée pour pulvériser un fluide de nettoyage vers la surface externe de la fenêtre d'observation.

9. Endoscope selon la revendication 8,
dans lequel l'unité de synchronisation est une unité de commande configurée pour réaliser une commande de pulvérisation du fluide depuis la buse d'injection de manière à être synchronisée avec l'opération de changement d'une portée du champ de visualisation d'imagerie de la partie d'imagerie par l'unité de déplacement de partie d'imagerie.

10. Endoscope selon la revendication 2,
dans lequel l'unité de nettoyage comprend une buse d'injection configurée pour pulvériser un fluide de nettoyage vers la surface externe de la fenêtre d'observation, et
l'unité de changement de position de nettoyage change une position de nettoyage par déplacement d'une direction de la buse d'injection.

11. Endoscope selon la revendication 2,
dans lequel l'unité de nettoyage comprend une buse d'injection configurée pour pulvériser un fluide de nettoyage vers la surface externe de la fenêtre d'observation, et
l'unité de changement de position de nettoyage présente une plaque réfléchissante configurée pour réfléchir le fluide de nettoyage qui est pulvérisé depuis la buse d'injection vers la surface externe de la fenêtre d'observation, et change une position de nettoyage à l'aide du fluide de nettoyage par déplacement d'une direction de la plaque réfléchissante.
